# EUROPEAN PATENT APPLICATION

(11) **EP 4 484 550 A1**
(43) Date of publication of application: **01.01.2025**
(21) Application number: 23868458.3
(22) Date of filing: 06.09.2023
(51) Int. Cl.: C12N 1/20, C12N 9/64, C07K 14/33

(54) **METHOD FOR INCREASING BOTULINUM TOXIN PRODUCTIVITY**

(30) Priority: 23.09.2022 KR 20220120506
(71) Applicant: JETEMA CO., LTD., Gangwon-do 26355 (KR)
(72) Inventor: KIM, Jae Young, Seoul 05649 (KR); NAM, Jeong Sun, Seoul 04425 (KR); YUN, Bum Jin, Seoul 05502 (KR); KIM, Seungho, Seoul 03491 (KR); KIM, Minjoong, Suwon-si, Gyeonggi-do 16267 (KR); CHEON, Sujin, Incheon 22765 (KR); LEE, Do Hee, Ansan-si, Gyeonggi-do 15628 (KR)
(74) Representative: Crow, Martin
(86) International application number: PCT/KR2023/013355
(87) International publication number: WO 2024/063404

(57) **Abstract**

The present invention relates to a method of culturing a strain producing botulinum toxin for increasing botulinum toxin productivity, and a method of effectively producing botulinum toxin by culturing the strain by the method. The culture method of the present invention has the advantage of enabling commercial productivity by increasing botulinum toxin productivity 10-fold or more.

## Description

### [Technical Field]

The present invention relates to a method of increasing botulinum toxin productivity, and more particularly, to a method of culturing a strain producing botulinum toxin for increasing botulinum toxin productivity, and to a method of effectively producing botulinum toxin by culturing the strain by the method.

### [Background Art]

Botulinum toxin is a neurotoxin protein produced by bacteria such as Clostridium butyricum, Clostridium baratii and Clostridium botulinum. botulinum toxin blocks neuromuscular transmission and causes neuro-paralytic disease in humans and animals. Seven different types of botulinum toxin, A, B, C1, D, E, F, G, and H, have been identified, and each type can be distinguished by each type-specific antibody, and the severity of paralysis they cause and animal species they affect are different.

Botulinum consists of four groups, from groups I to IV. Groups I and II originate from human botulism, and group III originate from botulism in animals. On the other hand, group IV is not known to originate from botulism. Botulinum group I includes proteolytic types A, B, and F toxins, and botulinum group II includes non-proteolytic B, E, and F toxins. In addition, group I and group II can degrade gelatin, and group II can ferment various carbohydrates such as sucrose and mannose.

A molecular weight of the botulinum toxin protein molecule is about 150 kDa, consisting of a heavy chain of about 100 kDa conjugated to a light chain of about 50 kDa. However, botulinum toxin released by Clostridium bacteria is released as a complex between 150 kDa toxin and one or more non-toxin proteins. For example, botulinum toxin is released as 900 kDa, 500 kDa, and 300 kDa complexes.

Botulinum toxin can be fatal to humans, but recently, botulinum toxin has been developed for the purpose of treating various symptoms including neuromuscular disorders characterized by hyperactivity of skeletal muscles. For example, BOTOX^{®} is a trademark for botulinum toxin A commercially developed by Allergan, Inc., which is used for the treatment of blepharospasm, strabismus, cervical dystonia, and glabella (facial) wrinkles, and other serotypes are being researched to develop suitable uses for them and use them clinically.

Botulinum toxin for clinical use is generally isolated from a cell culture, and conventionally, botulinum toxin was isolated through culture, fermentation, and purification processes mainly using an animal-derived product. However, when botulinum toxin is produced using an animal-derived product, there is a concern that a patient may also be administered various animal-derived pathogens or infectious substances when administering botulinum toxin to the patient. For example, prions may be included in the produced botulinum toxin composition, here, prions are completely different types of infectious disease agents from bacteria, viruses, fungi, parasites, or the like., and when animals, including humans are infected, a hole is opened in the brain like a sponge, and nerve cells die, resulting in the loss of a corresponding brain function. The prions can generate abnormal conformational isoforms from the same nucleic acid sequences that make up normal proteins, and infectivity is present during a "recruitment reaction" in which normal isoforms become prion protein isoforms at the post-translational stage. This leads to the misfolding of normal endogenous cellular proteins into pathogenic prion structures. Creutzfeldt-Jakob disease is a rare neurodegenerative disease of transmissible spongiform encephalopathies, and an infectious agent thereof is an abnormal isoform of a prion protein. Individuals with Creutzfeldt-Jakob disease may develop akinetic mutism within 6 months of being healthy. Therefore, when administering a pharmaceutical composition including a biologic such as botulinum toxin obtained using an animal-derived product, there is a risk of acquiring a prion-mediated disease, such as Creutzfeldt-Jakob disease.

In order to eliminate the risks, attempts have been made to exclude animal-derived components from, for example, culture media in the process of producing botulinum toxin. Representatively, Allergan Inc. has devised a method of fermentation in a medium including soybean as a plant-derived protein to replace animal-derived components (Korean Patent Publication 10-2006-0102330), but there was a problem in that a strain had to be cultured for a long time to produce a sufficient amount of botulinum toxin.

Food allergy refers to a case caused by an immune response among abnormal reactions that occur after ingestion of food. In general, not only symptoms that cause many inconveniences in daily life, such as hives, angioedema, and atopic dermatitis, but also anaphylaxis in severe cases is dangerous enough to threaten life. The eight major causes announced by the FDA are milk, eggs, fish, shellfish, tree nuts, peanuts, wheat, and soybeans, and these eight causes account for 85-90% of the total. Therefore, there is a need for efforts to improve the productivity of botulinum toxin by effectively culturing a strain producing botulinum toxin while replacing a conventional animal-derived component medium and excluding possible allergens. Furthermore, in order to use botulinum toxin commercially, it is necessary to increase toxin productivity through a simple and economical process.

Accordingly, the present inventors have made diligent efforts to develop a method for culturing a strain producing botulinum toxin to increase botulinum toxin productivity, and as a result, the present invention was completed by confirming that botulinum toxin productivity can be effectively improved through pH adjustment and suitable medium composition during the culture process of a strain producing botulinum toxin.

### [Disclosure]

### [Technical Problem]

The present invention is directed to providing a method of increasing botulinum toxin productivity.

### [Technical Solution]

In order to achieve the above object, the present invention provides a method of culturing a strain producing botulinum toxin, including:
(a) culturing a strain producing botulinum toxin in a seed culture medium; and
(b) culturing the strain producing botulinum toxin for which the seed culture has been completed in a main culture medium of pH 5.0 to 6.0.

In the present invention, operation (a) may be characterized by culturing in an anaerobic environment.

In the present invention, the seed culture medium in operation (a) may include 3.0 to 4.0% (w/v) of potato peptone, 0.5 to 2.5 w/v% of yeast extract, and 0.5 to 2.5 w/v% of glucose as active components.

In the present invention, the seed culture medium in operation (a) may be subjected to gas replacement through a mixed gas including two or more gases selected from the group consisting of N₂, CO₂, and H₂.

In the present invention, the culturing in operation (a) may be performed at a temperature of 28 °C to 32 °C for 18 to 30 hours.

In the present invention, operation (b) may be characterized by culturing in an anaerobic environment.

In the present invention, the main culture medium in operation (b) may include 3.0 to 4.0% (w/v) of potato peptone, 0.5 to 2.5 w/v% of yeast extract, and 0.5 to 2.5 w/v% of glucose as active components.

In the present invention, the main culture medium in operation (b) may be prepared by titrating the seed culture medium to pH 6.8 to 7.2.

In the present invention, the main culture medium in operation (b) may be subjected to gas replacement through a mixed gas including two or more gases selected from the group consisting of N₂, CO₂, and H₂.

In the present invention, the culturing in operation (b) may be performed at a temperature of 28 °C to 32 °C for 24 to 144 hours.

In the present invention, the botulinum toxin may be one or more selected from the group consisting of type E botulinum toxin, non-proteolytic type B botulinum toxin, and non-proteolytic type F botulinum toxin.

The present invention also provides a medium composition for culturing a strain that produces one or more botulinum toxins selected from the group consisting of type E botulinum toxin, non-proteolytic type B botulinum toxin, and non-proteolytic type F botulinum toxin, including 3.0 to 4.0% (w/v) of potato peptone, 0.5 to 2.5 w/v% of yeast extract, and 0.5 to 2.5 w/v% of glucose as active components.

The present invention also provides a method of culturing a strain producing botulinum toxin, including:
(a) culturing the strain producing botulinum toxin by the method to produce botulinum toxin; and
(b) recovering the produced botulinum toxin.

### [Advantageous Effects]

When a strain producing botulinum toxin is cultured by the method according to the present invention, the growth rate of strains is significantly increased and a high yield of botulinum toxin can be obtained. Commercial productivity is possible by increasing botulinum toxin productivity 10-fold or more. In addition, a medium composition of the present invention free from animal-derived products and major allergens has an advantage of excellent safety. Furthermore, type E botulinum toxin has the advantage of being effective on the day of injection and lasting for up to one month, so that it can be used to manufacture biopharmaceuticals, tissue sealing and facial fixing lifting threads, lipolysis injections, and local analgesics.

### [Description of Drawings]

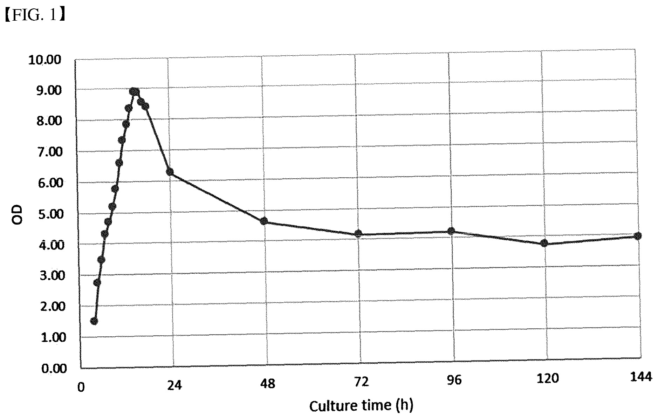

FIG. 1 illustrates changes in OD600 values of botulinum strains according to culture time.

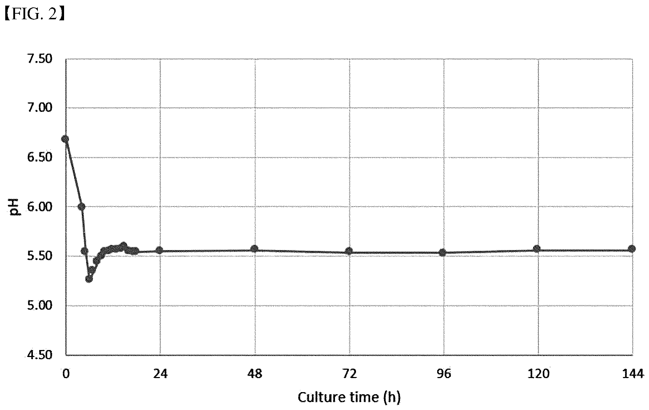

FIG. 2 illustrates a pH change of a culture fluid according to culture time;
FIG. 3 illustrates an amount of botulinum toxin according to culture time;
FIG. 4 illustrates a toxin amount according to a medium composition; and
FIG. 5 illustrate a toxin amount according to pH adjustment.
FIG. 6 illustrate a toxin amount according to pH adjustment.

### [Modes of the Invention]

Hereinafter, the present invention will be described in more detail.

All technical and scientific terms have the same meaning as commonly understood by one of ordinary skill in the art to which the present invention belongs. In general, the nomenclature used herein is well known and commonly used in the art.

In the present invention, the term "botulinum toxin" refers not only to a neurotoxin produced by Clostridium botulinum, but also to botulinum toxin (or a light chain or a heavy chain thereof) recombinantly produced by non-Clostridium-based species. As used herein, botulinum toxin also includes both pure botulinum toxin (that is, about 150 kDa) as well as botulinum toxin complexes (that is, 300, 600 and 900 kDa complexes). The "pure botulinum toxin" is defined as botulinum toxin that is isolated or substantially isolated from other proteins, including proteins that form the botulinum toxin complex. The pure botulinum toxin may have a purity of 95% or more, preferably 99% or more. Botulinum C2 and C3 cytotoxins are not neurotoxins and are excluded from the scope of the present invention.

In the present invention, the strain producing botulinum toxin may be Clostridium botulinum or a variant thereof, but is not limited thereto, and it will be apparent to those skilled in the art that any strain capable of producing botulinum toxin can be used.

Therefore, in one aspect, the present invention generally relates to a method of culturing a strain producing botulinum toxin, including:
(a) culturing a strain producing botulinum toxin in a seed culture medium; and
(b) culturing the strain producing botulinum toxin for which the seed culture has been completed in a main culture medium of pH 5.0 to 6.0, preferably pH 5.2 to 5.8, and more preferably pH 5.3 to 5.7.

In the present invention, operation (a) may be characterized by culturing in an anaerobic environment, but is not limited thereto.

In the present invention, a seed culture medium in operation (a) may include potato peptone, yeast extract, and glucose as active components, but is not limited thereto.

In the present invention, the potato peptone may be, without limitation, included at a concentration of 3.0 to 4.0% (w/v), preferably 3.3 to 3.8% (w/v), more preferably 3.5% (w/v), the yeast extract may be included at a concentration of 0.5 to 2.5 w/v %, preferably 1.0 to 1.5 w/v %, more preferably 1.2 w/v %, and the glucose may be included at a concentration of 0.5 to 2.5 w/v %, preferably 1.0 to 1.5 w/v%, more preferably 1.2 w/v%, but are not limited thereto.

In the present invention, the seed culture medium in operation (a) may be subjected to gas replacement through a mixed gas including two or more gases selected from the group consisting of N₂, CO₂ and H₂, but is not limited thereto.

In the present invention, the culturing in operation (b) may be performed at a temperature of 28 °C to 32 °C for 24 to 144 hours, preferably 48 to 96 hours, but is not limited thereto.

In the present invention, in order to maintain a pH range of 5.0 to 6.0, preferably pH 5.2 to 5.8, more preferably pH 5.3 to 5.7, after 4 hours of main culture, the culturing may be performed for 24 to 144 hours, preferably 48 to 96 hours while adjusting so as not to deviate from the pH range, but is not limited thereto. More specifically, titration is performed when the pH starts to deviate from the range, and then titration is performed until there is no change in pH. The pH titration may be performed with a basic substance, preferably sodium hydroxide, but is not limited thereto.

In the present invention, the pH decreases during main culture of strains, the pH reaches 5.3 after 4 to 8 hours of main culture, and the change in pH of a culture fluid slows down after 13 to 17 hours of main culture, so that the pH may be maintained around 5.5, but is not limited thereto. More specifically, pH adjustment may be started after 4 to 8 hours of main culture, and pH adjustment may be terminated when the change in pH of the culture fluid slows down after 13 to 17 hours of the main culture, but is not limited thereto.

In the present invention, the botulinum toxin may be botulinum toxin group II, but is not limited thereto.

The botulinum toxin group II may be one or more selected from the group consisting of type E botulinum toxin, non-proteolytic type B botulinum toxin, and non-proteolytic type F botulinum toxin, but is limited thereto.

In the present invention, the botulinum toxin productivity of strains can be increased 5-fold or more, preferably 9.5-fold or more, through the method, but is not limited thereto.

In another aspect, the present invention relates to a medium composition for culturing a strain that produces one or more botulinum toxins selected from the group consisting of type E botulinum toxin, non-proteolytic type B botulinum toxin, and non-proteolytic type F botulinum toxin including, as an active components, 3.0 to 4.0% (w/v), preferably 3.3 to 3.8% (w/v), more preferably 3.5% (w/v) of potato peptone, 0.5 to 2.5 w/v %, preferably 1.0 to 1.5 w/v %, more preferably 1.2 w/v % of yeast extract and 0.5 to 2.5 w/v %, preferably 1.0 to 1.5 w/v %, more preferably, 1.2 w/v% of glucose.

In another aspect, the present invention relates to a method of producing botulinum toxin, including.
(a) culturing a strain producing botulinum toxin by the method to produce botulinum toxin; and
(b) recovering the produced botulinum toxin.

In the present invention, the phrase "free of animal-derived components" means "substantially free of animal-derived components" or "substantially free of animal proteins", which means that there are no or substantially no blood-derived, blood-pooled and other animal-derived products or compounds. An "animal" means a mammal (such as a human), bird, reptile, fish, insect, spider or other animal species. The "animal" does not include a microorganism such as bacteria. Thus, animal product-free media or methods or substantially animal product-free media or methods within the scope of the present invention may include botulinum toxin or Clostridium-based botulinum bacteria. For example, the animal product-free or substantially animal product-free method means a method that is substantially, essentially, or completely free of animal-derived proteins such as immunoglobulins, meat digestion products, meat by-products, and milk or dairy products or digestion products. Therefore, examples of animal product-free methods include methods (such as bacterial culture or bacterial fermentation methods) that exclude meat and dairy products or meat or dairy by-products.

The present invention provides a medium including at least reduced levels of animal or dairy by-products, preferably substantially free of animal or dairy by-products. The "animal or dairy by-products" means a compound or a combination of compounds produced in or by animals (other than bacteria) cells, either in vivo or in vitro. Preferred non-animal sources of media components such as proteins, amino acids, and nitrogen include plants, microorganisms (such as yeast) and synthetic compounds.

The medium according to the present invention includes a medium for the growth and culture of Clostridium botulinum used to inoculate a medium for small- or large-scale fermentation of Clostridium botulinum, a seed medium (seed culture medium), and a fermentation medium (main culture medium), but is limited thereto.

As a particular preferred embodiment of the present invention, a medium for the growth of Clostridium botulinum and production of botulinum toxin may include a potato-derived component, preferably potato peptone, replacing an animal-derived component.

The present invention provides a method for the growth of Clostridium botulinum that maximizes the production of botulinum toxin in the shortest amount of time using a medium that is substantially free of animal-derived components. Clostridium botulinum can be grown using a medium composition in which animal-derived components are replaced with potato peptone.

In a preferred embodiment of the present invention, the growth of Clostridium botulinum proceeds in two operations (seed growth and fermentation). Both of these operations are preferably performed in an anaerobic environment. The seed growth (seed culture) operation is commonly used to "scale-up" an amount of microorganisms from a stored culture. The purpose of the seed growth (seed culture) operation is to increase the amount of microorganisms available for fermentation. Additionally, the seed growth (seed culture) operation allows relatively dormant microorganisms within the stored culture to rejuvenate and grow into actively growing cultures. Additionally, the volume and amount of viable microorganisms used to inoculate the fermentation medium can be more precisely controlled in more actively growing cultures in stored cultures. Therefore, the growth of seed cultures for inoculation into a fermentation medium (main culture medium) is preferred. It is also possible to use several consecutive operations involving growth in a seed medium (seed culture medium) to scale-up the amount of Clostridium botulinum for inoculation into a fermentation medium (main culture medium).

A fermentation (main culture) operation may include inoculating some of a seed medium (seed culture medium) including Clostridium botulinum from the seed growth operation or the entire seed medium (seed culture medium) into a fermentation medium (main culture medium). Preferably, about 1-10% of the seed culture (seed culture medium) having Clostridium botulinum from the seed growth (seed culture) operation is used to inoculate the fermentation medium (main culture medium). The fermentation (main culture) is used to produce the maximum amount of microorganisms in an anaerobic environment on a large scale.

The botulinum toxin included in the culture fluid of strains cultured by the method can be isolated and purified using a protein purification method known to those skilled in the art of protein purification.

The growth of Clostridium botulinum can proceed in one or more operations. Preferably, growth proceeds in two operations. In the first operation, seed growth (seed culture), Clostridium botulinum is suspended in the medium composition according to the present invention, and cultured for 18-30 hours at 30±2 °C under an anaerobic environment. Preferably, the seed growth (seed culture) proceeds for about 24 hours. It is also preferred that growth in the seed medium (seed culture medium) in any operation does not cause cell lysis prior to inoculation of a fermentation medium (main culture medium) with the final growth in the seed medium (seed culture medium).

Then, in order to further grow Clostridium botulinum and recover botulinum toxin, all or part of the seed growth (seed culture) medium is used to inoculate the medium composition of the present invention to proceed with the second operation, fermentation (main culture). After inoculation, incubation is performed for 48 to 96 hours at 30±2 °C under an anaerobic environment, and growth is monitored by measuring an optical density (OD) of the medium. Preferably, in the fermentation operation using the medium composition according to the present invention, after showing maximum growth between about 14 and 18 hours, cells are lysed and the OD value decreases, so that botulinum toxin is recovered within 96 hours, more preferably within 72 hours after starting the culture of Clostridium botulinum in the fermentation operation.

Hereinafter, the present invention will be described in more detail through examples. These examples are only for illustrating the present invention, and it will be apparent to those of ordinary skill in the art that the scope of the present invention is not to be construed as being limited by these examples.

### Examples

### Example 1

### Experimental materials

The experimental materials used in the present invention are as follows: purified water (Ultrapure water or water of equivalent quality), potato peptone E210 (Organotechnie, 19425), yeast extract (BD, 212750), Glucose (Merck, 1.37048.5000), sodium hydroxide pellet (Merck, 1.06482.5000), plastic syringes luer-lock 5 mL (Henke-Ject, 4050-X00V0), plastic syringes luer-lock 50 mL (Henke-Ject, 4850003000), 0.2 µm bottle top filter (Merck, S2GPT05RE), cellulose-based depth filter (Pall, NP6PDH41), cellulose acetate 0.2 µm filter (Pall, CSS92DPRRK).

### Example 2

### Preparation method of medium for culturing botulinum toxin-producing strain

500 mL of PYG medium (potato peptone 3.5%, yeast extract 1.2%, glucose 1.2%, and pH 7.0±0.2) was used as a seed culture medium. A main culture medium was prepared by titrating 5 L of the PYG medium to pH 7.0±0.2 using 5 N sodium hydroxide. After removing microorganisms with a 2 µm filter, the main culture medium was placed in a cell bag and attached to an incubator while paying attention to contamination, and used after gas replacement at 0.2 lpm for 4 hours or more using a mixed gas (N₂:CO₂:H₂ = 8:1:1).

### Example 3

### Method of culturing botulinum toxin-producing strain

### (Example 3-1) Strain preparation

1 vial of a Clostridium botulinum research cell bank (RCB) stored in a deep freezer was taken out. It was confirmed that the RCB was Clostridium botulinum type E NCTC8550. Thawing was performed in BSC for 10 minutes.

### (Example 3-2) Seed culture

In a biological safety cabinet, the thawed RCB was inoculated into a seed culture medium using a luer syringe while paying attention to contamination. An inoculated strain culture cell bag was placed in an anaerobic chamber substituted with a mixed gas set at 30 °C and cultured for 24 hours. After culturing for 24 hours, when the OD600 absorbance was 2 to 8, the seed culture was terminated.

### (Example 3-3) Main culture

In a biological safety cabinet, 100 mL of a culture fluid after the completion of seed culture was inoculated into a main culture medium using a luer syringe while paying attention to contamination. After inoculation, incubation was performed for 72 to 96 hours under the same conditions as in Table 1.

**[Table 1]**

| **Main culture conditions** | |
|---|---|
| **Conditions** | **Set point** |
| Temperature | 30±2 °C |
| Gas pressure | 0.2±0.1 lpm |
| Gas composition | N₂:CO₂:H₂=8:1:1 |

An OD600 value was measured by sampling a culture fluid after 4 hours of incubation. If the absorbance of the culture fluid is 1 or more, the culture fluid is diluted 1/10 and measured. From the time when the pH dropped below 5.3, 1 N NaOH was connected to a cell bag and injected at a rate of 470 to 620 µL/min for 9 hours. It was checked whether the pH was maintained at 5.5 ± 0.2 until the end of the culture. The culture fluid was sampled every 24 h of culture and ELISA was performed. After incubation, bacteria were removed using a depth filter and 0.2 µm filter and it was stored at 4 °C

### Example 4

### Identification of botulinum toxin through enzyme-linked immunosorbent assay (ELISA)

Preparation was performed by diluting 5 µg/mL antibody A buffer: antibody A stock solution in ELISA plate-coating buffer, 100 µL was dispensed into each well of 96-well microplates and coating was performed for 16 hours or more, and then the process of washing each well with a wash buffer was repeated a total of three times. The process of blocking each well with a blocking buffer (Super block) and washing each well with a wash buffer was repeated a total of three times.

after diluting a culture fluid sampled during culture, 100 µL was added to each well, reacted for 2 hours, and then washed with a wash buffer a total of three times. 100 µL of a detection antibody dilution solution was added to each well, reacted for 2 hours, and then washed with a wash buffer a total of three times. 100 µL of an anti-IgG antibody dilution solution was added to each well, reacted for 1 hour, and then washed with a wash buffer a total of five times. After adding 100 µL of TMB to each well and reacting for 20 minutes, 50 µL of a stop solution was added to each well, and an enzyme reaction was stopped by shaking appropriately. Absorbance at 450 nM was measured in each well using a microplate reader.

### Example 5

### Comparison of botulinum toxin production according to medium composition

The present inventors used a medium composition (potato peptone: 3%, yeast extract: 1%, and glucose: 1%) disclosed in a prior patent (Korean Patent Publication No. 10-2020-0114722) and a medium composition proposed in the present invention (potato peptone: 3.5%, yeast extract: 1.2%, and glucose: 1.2%), each type E botulinum toxin-producing strain was cultured, and amounts of botulinum toxin produced were compared.

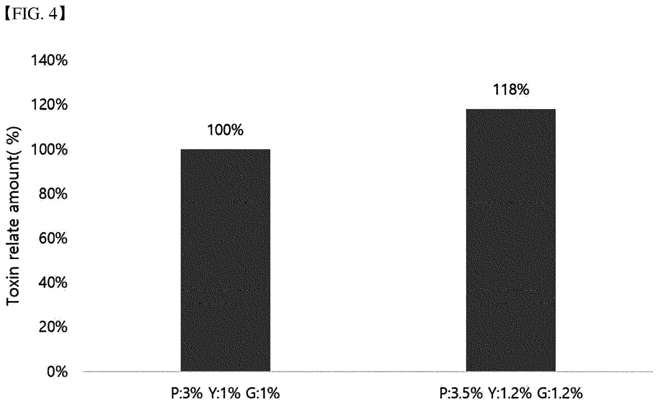

As a result, it was confirmed that when the strain was cultured in the medium composition of the present invention, a toxin amount increased about 18% compared to when the strain was cultured in the medium composition of the prior patent (FIG. 4). That is, it was confirmed that toxin production was excellent when a strain producing type E botulinum toxin was cultured in a medium including potato peptone: 3.5%, yeast extract: 1.2%, and glucose: 1.2%. Thus, it was confirmed that a medium composition for culturing a strain producing type E botulinum toxin and a medium composition for culturing a strain producing type A botulinum toxin were different.

### Example 6

### Comparison of botulinum toxin production according to pH titration

After culturing as before, pH titration conditions of a culture fluid were set as shown in Table 2, and during a culture process, culture was performed while the pH was adjusted so as not to deviate from a set range.

**[Table 2]**

| **pH titration conditions during culture** | |
|---|---|
| **Test group** | **Conditions** |
| Control | No pH titration (existing batch) |
| pH 6.0 test group | Adjust pH 6.0±0.2 during culture |
| pH 5.5 test group | Adjust pH 5.5±0.2 during culture |

As a result of the culture, pH titration was not performed in the control, which is an existing batch, so the pH was confirmed to be 4.85 at the time of final recovery, and the pH 6.0 test group and the pH 5.5 test group were terminated with a pH within the above-adjusted range. Specifically, at the final recovery time, the pH 6.0 test group was confirmed as pH 5.8 to 6.2, and the pH 5.5 test group was confirmed as pH 5.3 to 5.7.

**[Table 3]**

| **pH titration results during culture** | | | | |
|---|---|---|---|---|
| **Test group** | **Max OD** | **Final OD** | **Autolysis** | **Toxin (mg/L)** |
| Control | 5.74 | 4.79 | 17 % | 1.90 |
| pH 6.0 test group | 7.54 | 2.57 | 66% | 9.77 |
| pH 5.5 test group | 6.01 | 3.66 | 39 % | 18.10 |

When comparing three batches cultured under the conditions in Table 2 above, the pH 6.0 test group titrated to pH 6.0 had the highest strain maximum OD value and autolysis percentage, and the control without pH titration had the lowest. These results support that proper pH adjustment is necessary for the growth of strains.

In addition, when comparing toxin amounts in three batches in Table 3, it was confirmed that the pH 5.5 test group titrated to pH 5.5±0.2 had the highest level of 18.10 mg/L, showing excellent productivity. Therefore, an experiment was performed after setting appropriate conditions to pH 5.5±0.2.

### Example 7

### Comparison of botulinum toxin production according to culture time

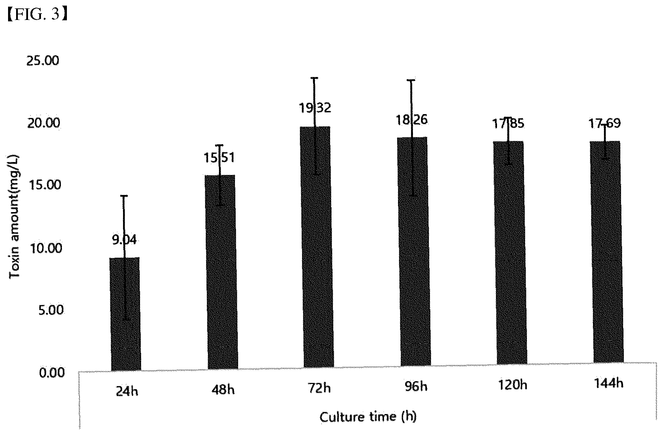

When a toxin amount in a culture fluid was measured on a 24-hour basis, the toxin amount was similar after 48 hours (FIG. 3). After 48 hours, it was confirmed that the toxin amount was similar, so that the culture time was set to 48 to 96 hours.

### Example 8

### Comparison of botulinum toxin production according to culture conditions

Culture condition parameters were set through a DOE experimental design. Results of culturing under the above culture conditions and measuring a toxin amount are shown in Table 4.

**[Table 4]**

| **Measurement result of toxin amount according to DOE experimental design** | | | | | | |
|---|---|---|---|---|---|---|
| **Block** | **Batch no.** | **Temp.** | **Titration pH** | **Gas pressure** | **Gas type** | **Toxin amount (mg/L)** |
| 1 | DOE 1 | 32 | 5.2 | 100 | Mixed | 6.95 |
| | DOE 2 | 28 | 5.8 | 300 | Nitrogen | 18.15 |
| | DOE 3 | 30 | 5.5 | 200 | Mixed | 27.64 |
| 2 | DOE 4 | 32 | 5.8 | 100 | Nitrogen | 18.61 |
| | DOE 5 | 28 | 5.2 | 300 | Mixed | 1.50 |
| | DOE 6 | 30 | 5.5 | 200 | Nitrogen | 15.61 |
| 3 | DOE 7 | 28 | 5.8 | 100 | Mixed | 16.64 |
| | DOE 8 | 32 | 5.2 | 300 | Nitrogen | 8.07 |
| | DOE 9 | 30 | 5.5 | 200 | Mixed | 25.04 |
| 4 | DOE 10 | 28 | 5.2 | 100 | Nitrogen | 7.345 |
| | DOE 11 | 32 | 5.8 | 300 | Mixed | 11.65 |
| | DOE 12 | 30 | 5.5 | 200 | Nitrogen | 17.09 |

As a result of analyzing a toxin amount in Table 4 through a statistical program (Minitab18), the factor influencing the toxin amount under the set conditions was confirmed by a pH titration value (Table 5). A culture temperature, a gas pressure, and a gas type did not have a significant effect on the toxin amount under the set conditions.

It was confirmed that the P-value was 0.016 under the pH 5.5±0.3 condition. That is, it was confirmed that the P-value of pH was smaller than 0.05, there was no confounding between the variables, and a curvature effect was present.

**[Table 5]**

| Screening results for factors affecting toxin amount | | |
|---|---|---|
| | Coef | P |
| Constant | 55.57 | |
| Temp. | 1.02 | 0.904 |
| pH | 25.75 | 0.016 |
| Gas pressure | -6.36 | 0.461 |
| Gas type | | |
| Temp.*pH | | |
| Temp.*Gas pressure | | |
| Temp.*Gas type | | |
| Ct Pt | 51.2 | 0.008 |
| S | | 23.0441 |
| R-sq | | 77.24 % |
| R-sq(adj) | | 64.24 % |
| R-sq(pred) | | 44.43 % |
| AICc | | 131.68 |
| BIC | | 117.79 |
| α to remove = 0.05 | | |

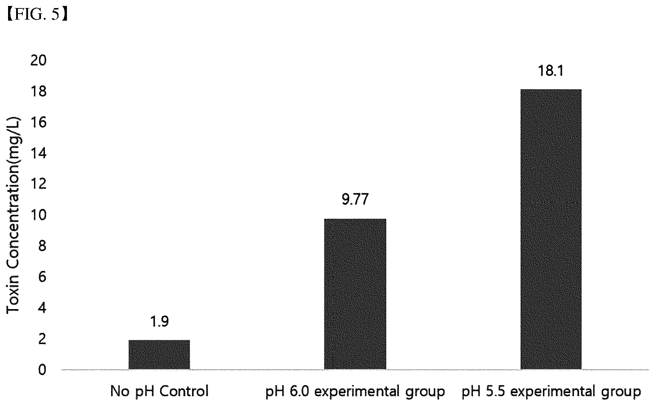

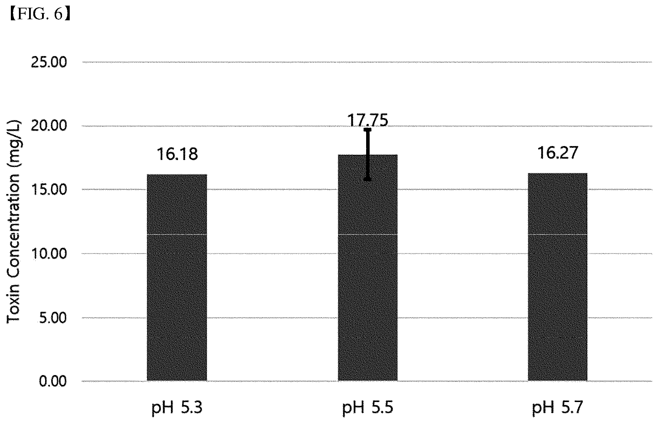

Specifically, as a result of comparing toxin amounts in Table 4, it was confirmed that a toxin amount was the highest in a midpoint batch titrated to pH 5.5. It was confirmed that a significant difference occurred in a toxin amount depending on the presence or absence of pH titration and conditions during culture (FIG. 5). In addition, it was confirmed that the pH 5.5±0.2 condition during culture showed similar productivity to the pH 5.5 culture condition (FIG. 6). Therefore, pH was set to 5.5 ± 0.2 (FIG. 6).

The culture temperature was found to have no significant effect on the toxin amount within the range of 28 to 32 °C and was therefore set at 30 ± 2 °C (Table 5).

The gas pressure was set to 200±100 lpm as it was confirmed that there was no significant effect on the toxin amount within the range of 100 to 300 lpm (Table 5).

As for the type of gas, it was confirmed that neither the mixed gas nor nitrogen gas had a significant effect on the toxin amount, so that it was judged that both types of gas could be used (Table 5). However, as a result of confirming the toxin amount (Table 4), in the midpoint condition, the mixed gas-incubated DOE 3 and DOE 9 batches was found to have approximately 38% higher average toxin amount (26.34, 16.35 mg/L) than the nitrogen gas-incubated DOE 6 and DOE 12 batches. Therefore, both the mixed gas and the nitrogen gas can be used when culturing strains, but use of the mixed gas is recommended.

## Claims

1. A method of culturing a strain producing botulinum toxin, comprising:
(a) culturing a strain producing botulinum toxin in a seed culture medium; and
(b) culturing the strain producing botulinum toxin for which the seed culture has been completed in a main culture medium of pH 5.0 to 6.0.

2. The method of claim 1, wherein the culturing in operation (a) is performed in an anaerobic environment.

3. The method of claim 1, wherein the seed culture medium in operation (a) includes 3.0 to 4.0% (w/v) of potato peptone, 0.5 to 2.5 w/v% of yeast extract, and 0.5 to 2.5 w/v% of glucose as active components.

4. The method of claim 1, wherein the seed culture medium in operation (a) is subjected to gas replacement through a mixed gas including two or more gases selected from the group consisting of N₂, CO₂, and H₂.

5. The method of claim 1, wherein the culturing in operation (a) is performed at a temperature of 28 °C to 32 °C for 18 to 30 hours.

6. The method of claim 1, wherein the culturing in operation (b) is performed in an anaerobic environment.

7. The method of claim 1, wherein the main culture medium in operation (b) includes 3.0 to 4.0% (w/v) of potato peptone, 0.5 to 2.5 w/v% of yeast extract, and 0.5 to 2.5 w/v% of glucose as active components.

8. The method of claim 1, wherein the main culture medium in operation (b) is prepared by titrating the seed culture medium to pH 6.8 to 7.2.

9. The method of claim 1, wherein the main culture medium in operation (b) is subjected to gas replacement through a mixed gas including two or more gases selected from the group consisting of N₂, CO₂, and H₂.

10. The method of claim 1, wherein the culturing in operation (b) is performed at a temperature of 28 °C to 32 °C for 24 to 144 hours.

11. The method of claim 1, wherein the botulinum toxin is one or more selected from the group consisting of type E botulinum toxin, non-proteolytic type B botulinum toxin, and non-proteolytic type F botulinum toxin.

12. A medium composition for culturing a strain that produces one or more botulinum toxins selected from the group consisting of type E botulinum toxin, non-proteolytic type B botulinum toxin, and non-proteolytic type F botulinum toxin, comprising 3.0 to 4.0% (w/v) of potato peptone, 0.5 to 2.5 w/v% of yeast extract, and 0.5 to 2.5 w/v% of glucose as active components.

13. A method of producing botulinum toxin, comprising:
(a) culturing the strain producing botulinum toxin by the method of any one of claims 1 to 11 to produce botulinum toxin; and
(b) recovering the produced botulinum toxin.
